(19) Europäisches Patentamt
European Patent Office
Office européen des brevets

(11) **EP 0 726 256 A1**

(12) **EUROPEAN PATENT APPLICATION**

(43) Date of publication:
**14.08.1996 Bulletin 1996/33**

(51) Int. Cl.[6]: **C07D 243/02**, C07D 491/04, C07D 405/06, A61K 31/55

(21) Application number: **96101883.5**

(22) Date of filing: **09.02.1996**

(84) Designated Contracting States:
**AT CH DE DK GR LI NL SE**

(30) Priority: **09.02.1995 HU 9500386**
**24.11.1995 HU 9503353**
**09.02.1995 HU 9500385**

(71) Applicant: **EGIS GYOGYSZERGYAR RT.**
**H-1106 Budapest (HU)**

(72) Inventors:
- **Vágo, Pál, Dr.**
  **H-1173 Budapest (HU)**
- **Reiter, Jozsef, Dr.**
  **H-1022 Budapest (HU)**
- **Gyertyán, István, Dr.**
  **H-1165 Budapest (HU)**
- **Gigler, Gábor**
  **H-1067 Budapest (HU)**
- **Andrási, Ferenc, Dr.**
  **H-1125 Budapest (HU)**
- **Bakonyi, Anna, Dr.**
  **H-1138 Budapest (HU)**
- **Berzsensyi, Pál, Dr.**
  **H-1174 Budapest (HU)**
- **Botka, Péter**
  **Deceased (HU)**
- **Birkás Faiglne, Erzsébet, Dr.**
  **H-1102 Budapest (HU)**
- **Hámori, Tamás, Dr.**
  **H-1031 Budapest (HU)**
- **Horváth, Edit, Dr.**
  **H-1119 Budapest (HU)**
- **Horváth, Katalin, Dr.**
  **H-1026 Budapest (HU)**
- **Korösi, Jeno, Dr.**
  **H-1013 Budapest (HU)**
- **Máté, Györgyné, Dr.**
  **H-1046 Budapest (HU)**
- **Moravcsik, Imre**
  **H-1095 Budapest (HU)**
- **Somogyi, Györgyi**
  **H-1148 Budapest (HU)**
- **Szentkuti, Eszter, Dr.**
  **H-1165 Budapest (HU)**
- **Zolyomi, Gábor, Dr.**
  **H-1184 Budapest (HU)**

(74) Representative: **Beszédes, Stephan G., Dr.**
**Patentanwalt**
**Postfach 11 68**
**85201 Dachau (DE)**

(54) **1-Arylvinyl-3,4-dihydro-5H-2,3-benzodiazepine derivatives useful for the treatment of central nervous system disorders**

(57) 2,3-Benzodiazepine derivatives of general formula

EP 0 726 256 A1

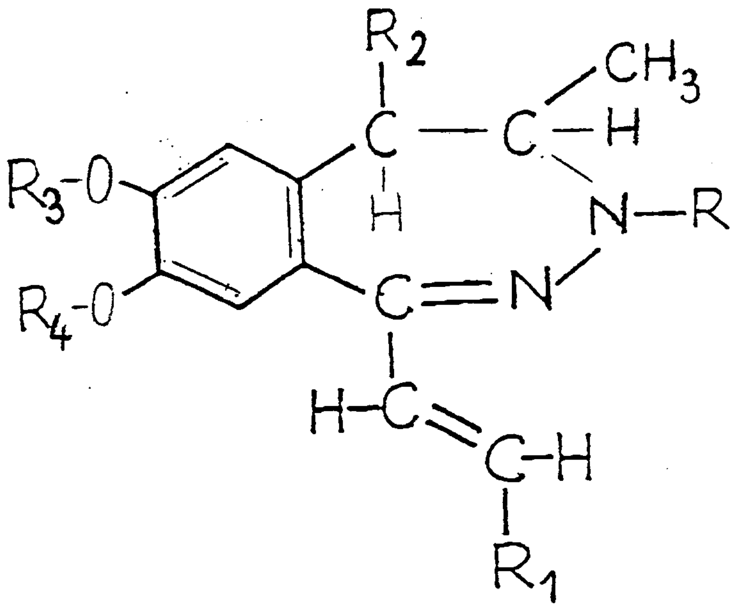

I,

wherein

| | |
|---|---|
| R | represents hydrogen or $C_{1-4}$-alkanoyl, |
| $R_1$ | stands for phenyl, optionally carrying 1 to 3 identical or different substituent(s) selected from the group consisting of halogen, nitro, amino, $C_{1-4}$-alkylamino,($C_{1-4}$-alkyl)-amino, $C_{1-4}$-alkanoylamino, $C_{1-4}$-alkyl, $C_{1-4}$-alkoxy, methylenedioxy and hydroxy; or naphthyl, optionally carrying a substituent selected from the group consisting of hydroxy, $C_{1-4}$-alkyl and $C_{1-4}$-alkoxy; |
| $R_2$ | stands for hydrogen or $C_{1-4}$-alkyl; |
| $R_3$ and $R_4$ | independently represent $C_{1-4}$-alkyl or |
| $R_3$ and $R_4$ | together form methylene, |

as well as acid addition salts and stereoisomers and mixtures thereof, and their use for preparing medicaments, particularly for the treatment of central nervous system disorders.

## Description

This invention is concerned with new 1-[2'-(substituted) vinyl]-3,4-di-[hydro]-5H-2,3-benzodiazepine derivatives with useful pharmacological effects, particularly on central nervous system disorders, a process for preparing them as well as medicaments containing these compounds and the use of them.

So far 3,4-di-[hydro]-5H-2,3-benzodiazepine derivatives containing a hydrogen atom or a methyl, phenyl, naphthyl, substituted phenyl, furyl or thienyl substituent at the 1 position of the basic molecule skeleton have been described (Hungarian patent specifications Nos. 168,760, 198,494 and 206,791, Hungarian patent application No. 8398/90, Chem. Ber. 95, 2 012 [1962]; Helv. Chim. Acta 59, 2 786 [1976]; Synthesis 1973, 159 and 1977, 1; Acta Chim. Hung. 83, 115 [1974]; Rec. Trav. Chim. 84, 661 [1965]; J. Chem. Soc. Chem. Comm. 1972, 823; Il Farmaco-Ed. Sc. 40, 942 [1985]; Chem. Pharm. Bull. 30, 3 764 [1982].

The known 1-[4'-(amino)-phenyl]-4-[methyl]-7,8-[methylenedioxy]-5H-2,3-benzodiazepine (the compound referred to as GYKI-52466) is a non-NMDA-glutamic acid antagonist having spasmolytic and antiischemic activities, but the duration of its action is rather short, and this fact represents a disadvantage in the therapeutic applicability thereof.

Hence the problem underlying to the invention is to create novel substituted 3,4-di-[hydro]-5H-2,3-benzodiazepine derivatives showing valuable pharmacological properties, particularly for the treatment of central nervous system disorders, comparable to those of the hitherto known benzodiazepines, but superior to them in view of the duration of the activity, a process for preparing them as well as medicaments containing these compounds and the use of them.

Surprisingly this has been solved by the invention.

Thus a subject-matter of the invention are 1-[2'-(substituted) vinyl]-3,4-di-[hydro]-5H-2,3-benzodiazepine derivatives of general formula

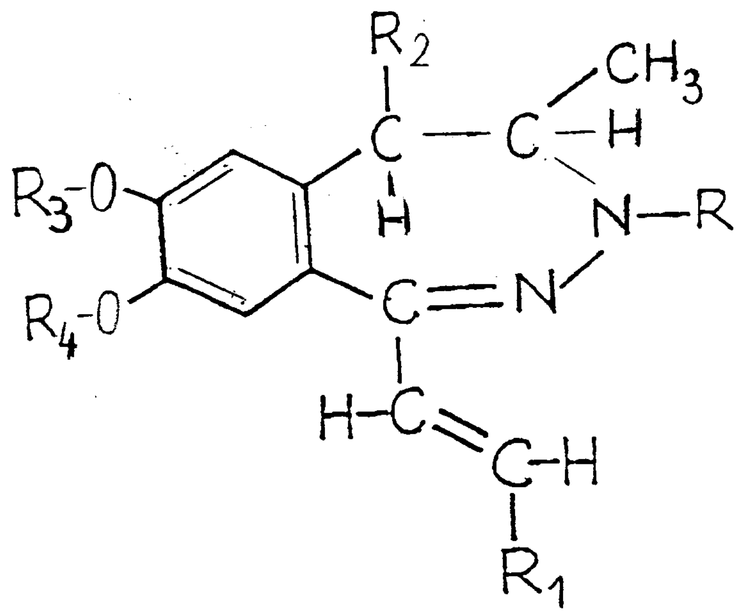

I,

wherein

| | |
|---|---|
| R | represents hydrogen or $C_{1-4}$-alkanoyl, |
| $R_1$ | stands for phenyl, optionally carrying 1 to 3 identical or different substituent(s) selected from the group consisting of halogen, nitro, amino, $C_{1-4}$-alkylamino, di-($C_{1-4}$-alkyl)-amino, $C_{1-4}$-alkanoylamino, $C_{1-4}$-alkyl, $C_{1-4}$-alkoxy, methylenedioxy and hydroxy; or naphthyl, optionally carrying a substituent selected from the group consisting of hydroxy, $C_{1-4}$-alkyl and $C_{1-4}$-alkoxy; |
| $R_2$ | stands for hydrogen or $C_{1-4}$-alkyl; |
| $R_3$ and $R_4$ | independently represent $C_{1-4}$-alkyl or |
| $R_3$ and $R_4$ | together form methylene, |

as well as acid addition salts and stereoisomers and mixtures thereof.

The term "alkyl" refers to straight or branched chained ones having the given number of carbon atom(s), such as methyl, ethyl, n-propyl or isopropyl. The term "alkoxy" relates to straight or branched chained alkyl ether groups, such as methoxy, ethoxy or isopropoxy. The term "alkanoylamino" relates to straight or branched chained aliphatic carboxylic acid amide groups, e.g. acetylamino or propanoylamino. The term "halogen" encompasses all the four halogen atoms, such as fluorine, chlorine, iodine and bromine.

Preferably the alkyl and/or alkoxy group(s) by which the phenyl group by which $R_1$ may be represented may be substituted or the alkyl or alkoxy group by which the naphthyl group by which $R_1$ may be represented may be substituted is/are such having from 1 to 3, particularly 1 or 2, most particularly 1, carbon atom(s).

It is also preferred that the alkyl part(s) of the alkylamino and/or di-(alkyl)-amino group(s) by which the phenyl group by which $R_1$ may be represented may be substituted is/are such having from 1 to 3, particularly 1 or 2, most particularly 1, carbon atom(s).

Furthermore it is preferred that the alkanoylamino group(s) by which the phenyl group by which $R_1$ may be represented may be substituted and/or which may be represented by R is/are such having from 1 to 3, particularly 2 or 3, most particularly 2, carbon atom(s).

Moreover it is preferred that the halogen atom(s) by which the phenyl group by which $R_1$ may be represented may be substituted is/are fluorine, bromine and/or chlorine, particularly chlorine.

Preferably the alkyl group which may be represented by $R_2$ is such having from 1 to 3, particularly 1 or 2, most particularly 2, carbon atom(s).

It is also preferred that the alkyl group(s) which may be represented by $R_3$ and/or $R_4$ is/are such having from 1 to 3, particularly 1 or 2, most particularly 1, carbon atom(s).

In case $R_1$ stands for phenyl carrying more than 1 substituent preferred groups are 2,3-di-(methoxy)-phenyl, 3,4-di-(methoxy)-phenyl, 2,4-di-(methoxy)-phenyl, 3-(ethoxy)-4-(hydroxy)-phenyl, 2,4,6-tri-(methoxy)-phenyl, 3-(isopropyl)-4-(methoxy)-phenyl and 3,4-di-(chloro)-phenyl.

A preferred group of 1-[2'-(substituted) vinyl]-3,4-di-[hydro]-5H-2,3-benzodiazepine derivatives according to the invention are those in which

| | |
|---|---|
| R | represents $C_{1-4}$-alkanoyl, |
| $R_1$ | stands for phenyl or naphthyl carrying a $C_{1-4}$-alkanoylamino or $C_{1-4}$-alkoxy substituent, |
| $R_2$ | stands for hydrogen or ethyl and |
| $R_3$ and $R_4$ | independently represent $C_{1-4}$-alkyl, |

as well as acid addition salts and stereoisomers and mixtures thereof.

Particularly preferred 1-[2'-(substituted) vinyl]-3,4-di-[hydro]-5H-2,3-benzodiazepine derivatives according to the invention are the following ones:

1-[4'-Acetylamino)-styryl]-3-[acetyl]-4-[methyl]-7,8-di-[methoxy]-3,4-di-[hydro]-5H-2,3-benzodiazepine, 1-[2'-(naphth-1"-yl)-vinyl]-4-[methyl]-7,8-di-[methoxy]-3,4-di -[hydro]-5H-2,3-benzodiazepine and 1-[2',3'-di-(methoxy)-styryl]-3-[acetyl]-4-[methyl]-7,8-di-[methoxy]-3,4-di-[hydro]-5H-2,3-benzodiazepine as well as acid addition salts and stereoisomers and mixtures thereof.

Suitably the acid addition salts of the 1-[2'-(substituted) vinyl]-3,4-di-[hydro]-5H-2,3-benzodiazepine derivatives of formula I according to the invention are pharmaceutically acceptable ones. Advantageous acid addition salts of the 1-[2'-(substituted) vinyl]-3,4-di-[hydro]-5H-2,3-benzodiazepine derivatives of formula I according to the invention are those with inorganic acids, e.g. hydrogenhalides, such as hydro-chloric acid or hydrobromic acid, sulfuric acid, phosphoric acid or perhaloacids, such as perchloric acid, or organic carboxylic acids, e.g. fumaric, acetic, propionic, glycolic, maleic, hydroxymaleic, ascorbinic, citric, malic, salicylic, lactic, cinnamic, benzoic, phenylacetic, p-aminobenzoic, p-hydroxybenzoic or p-aminosalicylic acid, alkylsulfonic acids, e.g. methanesulfonic or ethanesulfonic acid, or arylsulfonic acids, e.g. p-toluenesulfonic, p-bromophenylsulfonic, naphthylsulfonic or sulfanilic acid.

According to a further aspect of the present invention there is provided for a process for preparing the 1-[2'--(substituted) vinyl]-3,4-di-[hydro]-5H-2,3-benzodiazepine derivatives according to the invention, characterized in that

a) reducing 1-[2'-(substituted) vinyl]-5H-2,3-benzodiazepine derivatives of general formula

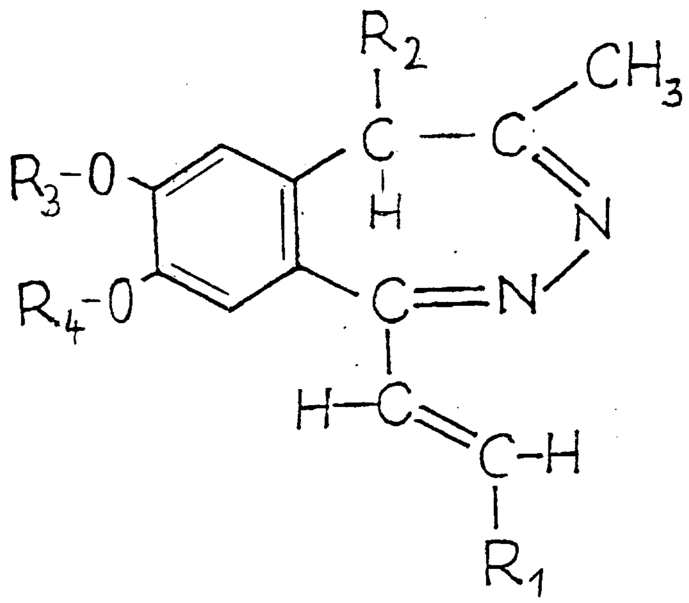

II,

wherein $R_1$ , $R_2$ , $R_3$ and $R_4$ are as above defined, with complex metal hydrides, boranes and/or borane complexes, and optionally in a manner known per se acylating the 1-[2'-(substituted) vinyl]-3,4-di-[hydro]-5H-2,3-benzodiazepine derivatives of general formula I thus obtained, wherein R stands for hydrogen and $R_1$ , $R_2$ , $R_3$ and $R_4$ are as above defined, or

b) in a manner known per se for preparing 1-[2'--(substituted) vinyl]-3,4-di-[hydro]-5H-2,3-benzodiazepine derivatives of general formula I, wherein $R_1$ represents aminophenyl, ($C_{1-4}$-alkyl)-aminophenyl, di-($C_{1-4}$-alkyl)-aminophenyl or ($C_{1-4}$-alkanoyl)-aminophenyl, which groups optionally carry 1 or 2 identical or different substituent(s) selected from the group consisting of halogen, nitro, amino, $C_{1-4}$-alkyl, $C_{1-4}$-alkoxy, methylenedioxy and hydroxy, and R, $R_2$ , $R_3$ and $R_4$ are as above defined, reducing 1-[2'-(substituted) vinyl]-3,4-di-[hydro]-5H-2,3-benzodiazepine derivatives of general formula I, wherein $R_1$ represents nitrophenyl, optionally carrying 1 or 2 identical or different substituent(s) selected from the group consisting of halogen, nitro, amino, methylenedioxy, $C_{1-4}$-alkyl, $C_{1-4}$-alkoxy and hydroxy, and R, $R_2$ , $R_3$ and $R_4$ are as above defined, with hydrazine hydrate in the presence of a catalyst, and optionally acylating or alkylating the thus obtained 1-[2'-(substituted) vinyl]-3,4-di-[hydro]-5H-2,3-benzodiazepine derivatives of general formula I, wherein $R_1$ stands for aminophenyl and R, $R_2$ , $R_3$ and $R_4$ are as above defined,

and optionally in a manner known per se converting the 1-[2'-(substituted) vinyl]-3,4-di-[hydro]-5H-2,3-benzodiazepine derivatives of general formula I obtained by any of the above variants a) and b) into acid addition salts thereof and/or optionally converting the acid addition salts of 1-[2'-(substituted) vinyl]-3,4-di-[hydro]-5H-2,3-benzodiazepine derivatives of general formula I obtained by any of the above variants a) and b) into the corresponding free1-[2'-(substituted) vinyl]-3,4-di-[hydro]-5H-2,3-benzodiazepine derivatives of general formula I and/or into other acid addition salts and/or resolving stereoisomer mixtures of the 1-[2'-(substituted) vinyl]-3,4-di-[hydro]-5H-2,3-benzodiazepine derivatives of formula I or acid addition salts thereof into their stereoisomers or converting the stereoisomers into mixtures thereof.

For the selective reduction of the 1-[2'-(substituted) vinyl]-5H-2,3-benzodiazepine derivatives of general formula II with complex metal hydrides, boranes and/or borane complexes according to variant a) of the process according to the invention advantageously the following reducing agents are used: sodium borohydride, lithium aluminum hydride, [a] borane(s) and/or [a] borane-dimethylamine complex(es). The reduction is preferably carried out in 1 or more solvent(s). Suitably the reduction of the 1-[2'-(substituted) vinyl]-5H-2,3-benzodiazepine derivatives of general formula II with complex metal hydrides, boranes and/or borane complexes is carried out in a solvent or solvent mixture, which does not interact with the applied reducing agent, or only very slowly. For this purpose advantageously water, 1 or more $C_{1-4}$-alcohol(s), $C_{1-4}$-carboxylic acid(s), solvent(s) of ether type, aromatic hydrocarbon(s), chlorinated aliphatic hydrocarbon(s) or pyridine or mixtures thereof is/are used. The solvents or solvent mixtures applicable in a given case depend on the applied reducing agent.

Suitably the reduction is carried out at a temperature of from 0°C to 100°C using preferably 1.1 to 25 molar equivalent(s) of reducing agent.

According to an embodiment of variant a) of the process according to the invention the starting 1-[2'-(substituted) vinyl]-5H-2,3-benzodiazepine derivatives of general formula II are dissolved or suspended in methanol, an excess of concentrated hydrochloric or acetic acid is added thereto, and sodium borohydride is added to the thus obtained hydrochloride or acetate. After working up the reaction mixture the desired 3,4-di-[hydro] compound, i.e. 1-[2'-(substituted) vinyl]-3,4-di-[hydro]-5H-2,3-benzodiazepine derivative of general formula I, is obtained by crystallization.

According to a preferred embodiment of variant a) of the process according to the invention 1.5 to 2.0 equivalents of borotrifluoride etherate are added to solutions or suspensions of the starting 1-[2'-(substituted) vinyl]-5H-2,3-benzodiazepine derivatives of general formula II in dry dichloromethane at a temperature of from 10°C to 15°C, to the solution of the thus obtained complex 1.1 equivalent of borane-trimethylamine complex, for example, compound no. 17, 898-5 of the Aldrich Catalogue, is added, and the reaction mixture is stirred at 25°C for 0.5 to 4 hour(s). The organic phase is then treated with sodium carbonate, washed with water, dried, evaporated, the desired product is crystallized, filtered and optionally recrystallized from a solvent, e.g. from a $C_{1-4}$-alcohol, or suspended in a solvent.

According to a further preferred embodiment of variant a) of the process according to the invention the 1-[2'-(substituted) vinyl]-5H-2,3-benzodiazepine derivatives are dissolved or suspended in anhydrous tetrahydrofurane, cooled to a temperature of from 0°C to 5°C, 1 molar equivalent of lithium aluminum hydride is added thereto, and the reaction mixture is stirred at room temperature for 2 hours. The complex is then decomposed, for example, with an aqueous potassium sodium tartrate solution, and the organic phase is evaporated. From the residue the desired 1-[2'-(substituted) vinyl]-3,4-di-[hydro]-5H-2,3-benzodiazepine of general formula I is obtained either by column chromatography or by crystallization.

For the reduction of the 1-[2'-(substituted) vinyl]-3,4-di-[hydro]-5H-2,3-benzodiazepine derivatives of general formula I, in which $R_1$ stands for a nitrophenyl group, optional-ly substituted as above defined, i.e. optionally substituted 1-[nitrostyryl]-3,4-di-[hydro]-5H-2,3-benzodiazepine derivatives, according to variant b) of the process according to the invention with hydrazine hydrate in the presence of a catalyst suitably a reduction method selective to the reduction of the nitro group is chosen, which does not saturate the vinyl group. So far no method has been provided in the literature for the reduction of such compounds. Now surprisingly it has been found that hydrazine hydrate applied in the presence of a catalyst is suitable for the selective reduction of compounds of such type. So far hydrazine hydrate applied in the presence of a catalyst has been used only for the conversion of nitro compounds containing no other reducible group into the corresponding amino compounds (Chem. Rev. 65, 52 [1965]; J. Am. Chem. Soc. 75, 4 334 [1953]; Chem. Lett. 1975, 259).

This reduction is preferably carried out in the presence of 1 or more organic solvent(s). Preferably the following solvents or mixtures thereof are used: 1 or more $C_{1-4}$-alcohol(s), dioxane, tetrahydrofurane, benzene, chloroform, dichloromethane, dimethylformamide, dimethyl sulfoxide and/or pyridine. It is preferable to carry out the reaction with an excess of 90 to 100% by weight hydrazine hydrate. As catalyst preferably palladium, particularly on bone charcoal, platinum or Raney nickel is used. Suitably the reduction is carried out at a temperature of from 0°C to the boiling point of the solvent or solvent mixture, preferably at a temperature of from +10°C to +100°C.

According to a preferred embodiment of variant b) of the process according to the invention the 1-(nitrostyryl)-5H-2,3-benzodiazepine derivatives, optionally substituted as above defined, of general formula II are suspended in methanol and reacted with 2 to 4 equivalents, preferably 3 equivalents, of 98 to 100% by weight hydrazine hydrate in the presence of Raney nickel catalyst at room temperature for 1 to 2 hour(s) and the crude product is separated from the reaction mixture by a method known per se. When the thus obtained product is hardly soluble in methanol, that is a partial separation occurs, it is preferable to wash the catalyst several times with a solvent or solvent mixture, wherein the product can be dissolved readily, such as chloroform. The crude product can be purified by recrystallization or trituration in a solvent. As solvent an alcohol or water or a mixture thereof can be used.

According to a further preferred embodiment of variant b) of the process according to the invention the 1-(nitrostyryl)-5H-2,3-benzodiazepine derivatives, optionally substituted as above defined, of general formula I are reacted for 1 to 2 hours with 3 equivalents of 100% by weight hydrazine hydrate in an alcohol at room temperature, a catalyst and further 2 equivalents of 100% by weight hydrazine hydrate are added to the reaction mixture and it is stirred further for a few hours at room temperature. The mixture is then worked up as specified above.

The optional alkylation of the 1-[2'-(substituted) vinyl]-3,4-di-[hydro]-5H-2,3-benzodiazepine derivatives of general formula I, in which $R_1$ stands for aminophenyl, thus obtained according to variant a) or b) of the process according to the invention can be performed by methods known per se, preferably with alkyl halides in 1 or more indifferent solvent(s), in the presence of 1 or more acid binding agent(s), at a temperature of from room temperature to the boiling point of the solvent(s). As solvent(s) preferably 1 or more aliphatic alcohol(s), ketone(s), nitrile(s), tetrahydrofurane, dioxane, dimethylformamide and/or dimethyl sulfoxide is/are used. As acid binding agent(s) preferably 1 or more alkali carbonate(s), alkali hydrogencarbonate(s) and/or 2 equivalent(s) of 1 or more $C_{1-4}$-tert.-amine(s) is/are used.

The optional acylation of the 1-[2'-(substituted) vinyl]-3,4-di-[hydro]-5H-2,3-benzodiazepine derivatives of the general formula I, in which R stands for hydrogen and/or $R_1$ represents aminophenyl, can be carried out by methods known in the literature, preferably with carboxylic acid halides or anhydrides. Suitably it is carried out by using 1 or 2 equivalent(s) of an acid halide or an acid anhydride. The reaction is preferably carried out in the presence of 1 or more acid

binding agent(s), particularly 1 or more aliphatic $C_{1-4}$-tert.-amine(s) and/or pyridine. It is preferable to carry out the reaction in 1 or more solvent(s), e.g. in [an] aliphatic ketone(s), nitrile(s), tetrahydrofurane, dioxane and/or pyridine, but the reaction can also be performed without using any solvent, in an excess of the applied reagent.

The 1-[2'-(substituted) vinyl]-5H-2,3-benzodiazepine derivatives of general formula II used as starting substances are new and can be prepared in a manner analogous to that described in Hungarian patent specification No. 195,788. The melting points of the new starting compounds are given below.

As already mentioned, the 1-[2'-(substituted) vinyl]-3,4-di-[hydro]-5H-2,3-benzodiazepine derivatives according to the invention possess valuable pharmaceutical properties, particularly central nervous activities.

Hence by the invention also there are provided for medicaments which are characterized in that they contain 1 or more 1-[2'-(substituted) vinyl]-3,4-di-[hydro]-5H-2,3-di-benzodiazepine derivative(s) according to the invention as [an] active ingredient(s), advantageously in admixture with 1 or more inert solid and/or liquid pharmaceutical carrier(s) and/or auxiliari(es).

The medicaments according to the invention can be in the form of pharmaceutical preparations. These can be prepared by methods known per se by admixing the 1 or more 1-[2'-(substituted) vinyl]-3,4-di-[hydro]-5H-2,3-benzodiazepine derivative(s) with 1 or more inert solid and/or liquid carrier(s) and/or auxiliari(es) and bringing the mixture to a galenic form.

Tablets, coated tablets, dragees and solid gelatin capsules can contain as carrier(s) e.g. lactose, corn starch, potato starch, talc, magnesium carbonate, magnesium stearate, calcium carbonate and/or stearic acid and/or [a] salt[s] thereof. Soft gelatin capsules can contain as carrier(s) e.g. [a] vegetable oil(s), fat(s), wax(es) and/or polyol(s) of suitable consistency. Solutions and syrups can contain as carrier(s) e.g. water, [a] polyol(s), such as polyethylene glycol, saccharose and/or glucose. The injection solutions can contain e.g. water, [an] alcohol(s), polyol(s), glycerol and/or [a] vegetable oil(s) as carrier(s). The suppositories can contain as carrier(s) e.g. [an] oil(s), wax(es), fat(s) and/or polyol(s) of suitable consistency.

In addition, the pharmaceutical preparations according to the invention may contain 1 or more auxiliari(es) usually applied in the pharmaceutical technique, e.g. [a] wetting agent(s), sweetening agent(s), aroma substance(s), salt(s) causing the change of osmotic pressure and/or buffer(s). They may further contain 1 or more other active ingredient(s), too.

The daily dose of the 1-[2'-(substituted) vinyl]-3,4-di-[hydro]-5H-2,3-benzodiazepine derivatives according to the invention can vary within wide ranges depending on several factors, e.g. on the activity of the active ingredient, the patient's condition and age and the severity of the disease. The preferred oral dose is generally 0.1 to 500 mg/day. It has to be stressed that the above dose is only of informative character and the administered dose must always be determined by the physician therapeutist.

According to a further aspect of the present invention there is provided for the use of the 1-[2'-(substituted) vinyl]-3,4-di-[hydro]-5H-2,3-benzodiazepine derivatives according to the invention for preparing medicaments, particularly for the treatment of central nervous system disorders.

The 1-[2'-(substituted) vinyl]-3,4-di-[hydro]-5H-2,3-benzodiazepine derivatives according to the present invention are to be administered to the patients in effective amounts.

The activity of the 1-[2'-(substituted) vinyl]-3,4-di-[hydro]-5H-2,3-benzodiazepine derivatives according to the invention has been proved by the following experiments.

As reference compound 1-(3'-(chloro)-phenyl)-4-(methyl)-7,8-di-(methoxy)-5H-2,3-benzodiazepine [girisopam] was used since this compound also binds to the homophthalazine binding site thus being a standard ligand of this binding site, and has central nervous system activities.

The 1-[2'-(substituted) vinyl]-3,4-di-[hydro]-5H-2,3-benzodiazepine derivatives according to the invention bind with high affinity to the binding site specific for homophthalazines (2,3-benzodiazepines) [FEBS Letters 308 (2), 215 to 217 (1992)] suggesting that assuming similar absorption and metabolism to those of 2,3-benzodiazepines - for this reason they will exert considerable in vivo activities in the central nervous system.

The binding affinity of 1-[2'-(substituted) vinyl]-3,4-di-[hydro]-5H-2,3-benzodiazepine derivatives according to the invention and of the reference substance to the homophthalazine binding site was measured by displacing 5nM of $^3$H-1-(3'-(chloro)-phenyl-4-(methyl)-7,8-di-(methoxy)-5H-2,3-benzodiazepine [$^3$H-girisopam] in a rat brain striatal membrane preparation. The $K_i$ values measured are shown in the following Table I. $K_i$ values were calculated using the following equation:

$$K_i = IC_{50} : \left(1 + \frac{[L]}{K_D}\right),$$

wherein $K_D$ is the dissociation constant of the labelled ligand-receptor complex, [L] is the concentration of the labelled ligand and $IC_{50}$ is the half maximal inhibitory concentration of the test compound.

Table I

| Compound (No. of Example | $K_i$(mole/l) |
|---|---|
| 5 | $7.85 \pm 0.51 \cdot 10^{-8}$ |
| 18 | $6.07 \pm 2.15 \cdot 10^{-8}$ |
| 21 | $3.13 \pm 0.74 \cdot 10^{-8}$ |
| 22 | $5.33 \pm 1.20 \cdot 10^{-8}$ |
| 1-(3'-(chloro)-phenyl-4-(methyl)-7,8-di-(methoxy)-5H-2,3-benzodiazepine [girisopam] {reference substance A} | $4.00 \cdot 10^{-8}$ |

The 1-[2'-(substituted) vinyl]-3,4-di-[hydro]-5H-2,3-benzodiazepine derivatives according to the invention considerably decrease the spontaneous motor activity (SMA) of mice after intraperitoneal or oral administration.

The spontaneous motor activity (SMA) inhibiting effect and the acute toxicity data (dead/treated animals, shown in brackets) of 1-[2'-(substituted) vinyl]-3,4-di-[hydro]-5H-2,3-benzodiazepine derivatives according to the invention and of the reference substance 1-(3'-(chloro)-phenyl-4-(methyl)-7,8-di-(methoxy)-5H-2,3-benzodiazepine [girisopam] are provided in the following Table II.

The experiments were performed according to the method of S. Irwin (Psychopharmacology 13, 222 [1968]).

Table II

| Compound (No. of Example) | Behaviour (acute toxicity) | | | | | |
|---|---|---|---|---|---|---|
| | p.o. 1000 mg/kg | I.p. 300 mg/kg | p.o. 300 mg/kg | I.p. 100 mg/kg | p.o. 100 mg/kg | I.p. 30 mg/kg |
| 1 | +-(0/5) | +-(0/5) | 0 | +- | 0 | 0 |
| 2 | + + | + + | + | +- | +- | 0 |
| 3 | ++(0/5) | + (0/5) | 0 | +- | 0 | 0 |
| 5 | +- | + + | +- | +- | +- | +- |
| 20 | + + | ++(1/5) | ++ | ++ | +- | +- |
| 22 | + + | ++(1/5) | + | + | +- | +- |
| 23 | + (0/5) | ++(0/5) | +- | +- | +- | +- |
| 26 | +-(0/5) | +-(0/5) | +- | +- | 0 | 0 |
| 27 | +- | +-(0/5) | +- | +- | +- | +- |
| 34 | + | + | + | + | + | 0 |
| 41 | +-(0/5) | +-(0/5) | +- | +- | +- | +- |
| 1-3'-(chloro)-phenyl-4-(methyl)-7,8-di-(methoxy)-5H-2,3-benzodiazepine [girisopam] {reference substance A} | | ++(0/6) | | +- | | 0 |
| Symbols: ++ strong, + medium, +- mild decrease of spontaneous motor activity [SMA], 0: no effect | | | | | | |

Contrary to the known compounds having similar chemical structure, the 1-[2'-(substituted) vinyl]-3,4-di-[hydro]-5H-2,3-benzodiazepine derivatives according to the invention show a significant potentiation of the stereotypy induced by 1-(phenyl)-propan-2-amine [amphetamine] suggesting an antidepressant activity. The potentiation of the stereotypy evoked by 1-(phenyl)-propan-2-amine [amphetamine] was evaluated according to the scale of Constall and Naylor (Eur. J. Pharmacol. 18, 95 [1972]). The results are shown in the following Table III.

Table III

| Compound (No. of Example) | Potentiation* % |
|---|---|
| 7 | 90.20 |
| 8 | 113.14 |
| 9 | 141.18 |
| 10 | 176.47 |
| 14 | 112.94 |
| 22 | 113.14 |
| 27 | 183.33 |
| 28 | 92.55 |
| 30 | 100 |
| 33 | 201.63 |
| 34 | 92.03 |
| 39 | 112.75 |
| 40 | 99.67 |

* 10 mg/kg i.p.+ 1-(Phenyl)-propan-2-amine [amphetamine] 3 mg/kg s.c.

The 1-[2'-(substituted) vinyl]-3,4-di-[hydro]-5H-2,3-benzodiazepine derivatives according to the invention exert a moderate anticonvulsive effect, too. This latter was measured in mice applying the method of Goodmann et al. (J. Pharmacol. Exp. Ther. 106, 319 [1952]). The convulsions evoked by 50 mg/kg i.v. of 6,7,8,9-tetra-(hydro)-5H-tetrazolo[1,5a]azepine {pentetrazole} were inhibited by 30 to 40% and 50 to 55% after the i.p. administration of 30 mg/kg of the 1-[2'-(substituted) vinyl]-3,4-di-[hydro]-5H-2,3-benzodiazepine derivatives of Examples 10, 11, 13, 19, 28, 35 and 40, and Examples 34 and 38, respectively.

The effects of the 1-[2'-(substituted) vinyl]-3,4-di-[hydro]-5H-2,3-benzodiazepine derivatives on the glutamatergic transmission were studied in hippocampal slices applying the method of Tarnawa et al. (Acta Physiol. Hung., 79, 163 [1992]. 400 µm thick slices were prepared from rat brain and maintained in a chamber of interface type under simulated psychological conditions. The Schaffer collaterals were stimulated and field potentials were recorded from the pyramidal cells of the hippocampal CA1 region. The neurotransmitter involved in this process is glutamate acting mainly through AMPA receptors. The known AMPA antagonist, the compound 1-[4'-(amino)-phenyl]-4-[methyl]-7,8-[methylenedioxy]-5H-2,3-benzodiazepine {GYKI-52466} concentration-dependently inhibits the CA1 field potentials. The results obtained are shown in the following Table IV.

Table IV

| Compound (No. of Example) | Concentration | Inhibition (%) 30 minutes after drug administration | Inhibition (%) 60 minutes after drug administration | Inhibition (%) after 30 minutes wash-out |
|---|---|---|---|---|
| 39 | 50μM | 85.3 ± 15.09 | 100 ± 0 | 97.5 ± 0.98 |
| 40 | 50μM | 14.8 ± 7.10 | 25.8 ± 3.04 | 35.4 ± 2,67 |
| 41 | 50μM | 79.5 ± 10.10 | 100 ± 0 | 98.6 ± 2.67 |
| 21 | 50μM | 16.1 ± 13.4 | 33.7 ± 7.60 | 61.1 ± 4.56 |
| 22 | 50μM | 4.7 ± 0.84 | 37.0 ± 9.19 | 74.3 ± 11.53 |
| 1-[4'-(amino)-phenyl]-4-[methyl]-7,8-[methylene-dioxy]-5H-2,3-benzodi-azepine {GYKI-52466} ⟨reference substance B⟩ | 50μM | 94.5 ± 2.07 | 100 ± 0 | 84.6 ± 7.37* |
| P < 0.05 compared to the 60 minutes value | | | | |

The 1-[2'-(substituted) vinyl]-3,4-di-[hydro]-5H-2,3-benzodiazepine derivatives of Examples 39 and 41 showed at least equal efficacy to that of 1-[4'-(amino)-phenyl]-4-[methyl]-7,8-[methylenedioxy]-5H-2,3-benzodiazepine {GYKI-52466} ⟨reference substance B⟩. In the case of the latter compound, however, the inhibition observed after a wash-out lasting for 30 minutes was significantly smaller than that observed after the preceding incubation period lasting for 60 minutes, while in the case of the 1-[2'-(substituted) vinyl]-3,4-di-[hydro]-5H-2,3-benzodiazepine derivatives of Examples 39 and 41 a wash-out lasting for 30 minutes was not able to diminish the effect. This means that the duration of action of the latter compounds surpasses that of 1-[4'-(amino)-phenyl]-4-[methyl]-7,8-[methylenedioxy]-5H-2,3-benzodiazepine {GYKI-52466} ⟨reference substance B⟩. In case of the further 3 test substances the inhibition observed after the wash-out period was even higher than that observed at the end of the preceding incubation period.

The inhibition of the hippocampal field potentials supports the possible antiischemic and neuroprotective therapeutical use of the 1-[2'-(substituted) vinyl]-3,4-di-[hydro]-5H-2,3-benzodiazepine derivatives according to the invention [Le Peillet et al.: Eur. J. Neurosci., 4 (suppl.), 1 068 [1991]. According to own results the duration of action of the 1-[2'-(substituted) vinyl]-3,4-di-[hydro]-5H-2,3-benzodiazepine derivatives according to the invention exceeds those of the known compounds having similar effect.

The present invention is further illustrated by means of the following Examples.

## Example 1

1-[3',4'-Di-(methoxy)-styryl]-4-[methyl]-7,8-[methylenedioxy]-3,4-di-[hydro]-5H-2,3-benzodiazepine

To a solution of 2.04 g (5.6 mmoles) 1-[3',4'-di-[methoxy)-styryl]-4-[methyl]-7,8-[methylenedioxy]-5H-2,3-benzodiazepine in 40 ml of anhydrous dichloromethane 1.0 ml (8.4 mmoles) of borotrifluoride etherate is added under cooling with tap water, and 0.45 g (6.16 mmoles) of borane trimethylamine complex (compound no. 17,898-5 of the Aldrich Catalogue) is added to it. The reaction mixture is stirred at 25°C for 0.5 hour, and then 30 ml of 10% by weight agueous sodium carbonate solution are dropped to it under cooling with tap water, and the mixture is stirred further for 1 hour. The organic phase is separated, washed four times with 30 ml each of distilled water, dried and evaporated. The crystalline residue is suspended in 10 ml of ethanol, filtered, washed three times with 1 ml each of ethanol and dried at a temperature between 80°C and 100°C. Thus 1.76 g of the desired product is obtained.

M.p.: 166 to 168°C.

In order to purify the crude product it is boiled in 10 ml of ethanol, cooled, filtered, washed three times with 1 ml each of ethanol and dried. Thus 1.69 g (82.4%) of the desired product is obtained.

M.p.: 168 to 170°C.

Further compounds of general formula I, wherein R represents hydrogen, have also been prepared according to the method of Example 1, which are summarized in the following Table V. In this Me = methyl and Et = ethyl and the % Et0H are by volume.

Table V

| Compound (No. of Example) | $R_1$ | $R_2$ | $R_3$ | $R_4$ | $BF_3$ etherate mole | M.p. (°C) | Cryst. | Yield |
|---|---|---|---|---|---|---|---|---|
| 2 | 3-(chloro)--phenyl | H | Me | Me | 1.5 | 105 to 107 | 80% *) EtOH | 61.0 |
| 3 | 3,4-(methylene-dioxy)-phenyl | H | Me | Me | 1.5 | 163 to 164 | EtOH *) | 76.5 |
| 4 | 4-(hydroxy)--phenyl | H | Me | Me | 1.5 | 160 to 162 | 50% *) EtOH | 43.0 |
| 5 | 3-(ethoxy)-4--(hydroxy)-phenyl | H | Me | Me | 1.5 | 138 to 139 | 50% *) EtOH | 50.0 |
| 6 | 2,4,6-tri--(methoxy)--phenyl | H | Me | Me | 1.5 | 144 to 146 | EtOH | 80.0 |
| 7 | 4-(dimethyl-amino)-phenyl | H | Me | Me | 2 | 145 to 147 | 50% *) EtOH | 76.6 |
| 8 | 3,4-(methylene-dioxy)-phenyl | H | $-CH_2-$ | | 1.5 | 181 to 183 | EtOH | 86,0 |
| 9 | 4-(dimethyl-amino)-phenyl | H | $-CH_2-$ | | 2 | 156 to 158 | EtOH | 76.2 |
| 10 | 4-(dimethyl-amino)-phenyl | H | Et | Et | 2 | 149 to 151 | EtOH | 78.6 |
| 11 | 3-(hydroxy)--phenyl | H | Me | Me | 1.5 | 106 to 108 | EtOH | 46.0 |

*) by volume

Table V continued

| Compound (No. of Example) | $R_1$ | $R_2$ | $R_3$ | $R_4$ | $BF_3$ etherate mole | M.p. (°C) | Cryst. | Yield |
|---|---|---|---|---|---|---|---|---|
| 12 | phenyl | H | Me | Me | 1.5 | 120 to 121 | EtOH | 65.0 |
| 13 | 3-(isopropyl)--4-(methoxy)--phenyl | H | $-CH_2-$ | | 1.5 | 149 to 151 | EtOH | 77.7 |
| 14 | 2-(bromo)--phenyl | H | Me | Me | 1.5 | 130 to 131 | EtOH | 50.0 |
| 15 | 3,4-di--(methoxy)--phenyl | Et | Me | Me | 1.75 | 157 to 158 | EtOH | 59.0 |

Example 16

1-[2',3'-Di-(methoxy)-styryl]-4-[methyl]-7,8-[methylenedioxy]-3,4-di-[hydro]-5H-2,3-benzodiazepine hydrochloride

1.5 g (4.12 mmoles) of 1-[2',3'-di-(methoxy)-styryl)-4-[methyl]-7,8-[methylenedioxy]-5H-2,3-benzodiazepine is reduced as specified in Example 1, the evaporation residue is dissolved in ethyl acetate and 10 ml of ethyl acetate saturated with 10% by weight gaseous hydrogen chloride are added to the solution. The separated product is filtered, washed three times with 5 ml each of ethyl acetate and dried at a temperature between 80°C and 100°C. Thus 0.76 g (45.8%) of the desired product is obtained. M.p.: 193 to 195°C (decomp.).

Example 17

1-[2',4'-Di-(methoxy)-styryl]-4-[methyl]-7,8-[methylenedioxy]-3,4-di-[hydro]-5H-2,3-benzodiazepine

A suspension of 1.1 g (3.0 mmoles) of 1-[2',4'-di-(methoxy)-styryl]-4-[methyl]-7,8-[methylenedioxy]-5H-2,3-benzodiazepine in 15 ml of anhydrous tetrahydrofurane is cooled to a temperature between 0°C and 5°C, and 0.114 g (3.0 mmoles) of lithium aluminum hydride is added to it. The reaction mixture is stirred at 25°C for 2 hours, cooled again to a temperature between 0°C and 5°C and decomposed with 0.36 ml of 10% by weight aqueous potassium sodium tartrate solution. Then it is stirred further for 1 hour at 25°C, the precipitate is filtered off, the filtrate is dried and evaporated under reduced pressure. The crude end-product is recrystallized from 10 ml of ethanol, filtered, washed three times with 1 ml each of ethanol and dried at a temperature between 80°C and 100°C. Thus 0.84 g (76.0%) of the desired product is obtained. M.p.: 176 to 178°C.

Example 18

1-[2',4'-Di-(methoxy)-styryl]-4-[methyl]-7,8-di-[methoxy]-3,4-di-[hydro]-5H-2,3-benzodiazepine

On starting from 1-[2',4'-di-(methoxy)-styryl]-4-[methyl]-7,8-di-[methoxy]-5H-2,3-benzodiazepine the procedure specified in Example 17 is followed, with the difference that the crude product obtained after the evaporation is purified by column chromatography [adsorbent: Kieselgel 60, particle size: 0.063-2 mm; eluent: benzene-methanol-cc. $NH_40H$ (ratio by volume = 8:2:0,1)]. The desired compound is obtained in crystalline form upon evaporating the fractions. Yield: 53%.
M.p.: 118 to 120°C.

Example 19

1-[2',4'-Di-(methoxy)-styryl]-3-[acetyl]-4-[methyl]-7,8-[methylenedioxy]-3,4-di-[hydro]-5H-2,3-benzodiazepine

The crude product obtained when evaporating the solution of the compound of Example 17 is dissolved in 7 ml of chloroform, 0.7 ml of acetic anhydride is added to it and the mixture is boiled for 2 hours. Then it is cooled to room temperature, 10 ml of water are added to it, and the mixture is adjusted to pH = 7 to 8 by the addition of sodium hydrogen carbonate. The organic phase is separated, the aqueous phase is extracted three times with 5 ml each of chloroform, the organic phases are combined and washed twice with 10 ml each of distilled water, dried and evaporated under reduced pressure. The evaporation residue is recrystallized from ethanol. Thus 0.8 g (65%) of the desired product is obtained. M.p.: 185 to 187°C.

The compounds of the following Examples 20 to 23 can be prepared according to the method of Example 19.

Example 20

1-[Styryl]-3-[acetyl]-4-[methyl]-7,8-di-[methoxy]-3,4-di-[hydro]-5H-2,3-benzodiazepine

Yield: 50,0%. M.p.: 118 to 120°C (Et0H).

Example 21

1-[2',3'-Di-(methoxy)-styryl]-3-[acetyl]-4-[methyl]-7,8-di-[methoxy]-3,4-di-[hydro]-5H-2,3-benzodiazepine

Yield: 56,0%. M.p.: 85 to 87°C (Et0H).

Example 22

1-[2',4'-Di-(methoxy)-styryl]-3-[acetyl]-4-[methyl]-7,8-di-[methoxy]-3,4-di-[hydro]-5H-2,3-benzodiazepine

Yield: 58,0%. M.p.: 72 to 74°C (Et0H).

Example 23

1-[2',3'-Di-(methoxy)-styryl]-3-[acetyl]-4-[methyl]-7,8-[methylenedioxy]-3,4-di-[hydro]-5H-2,3-benzodiazepine

Yield: 60,0%. M.p.: 125 to 128°C (Et0H).

Example 24

1-[4'-(Nitro)-styryl]-4-[methyl]-7,8-[methylenedioxy]-3,4-di-[hydro]-5H-2,3-benzodiazepine

To a suspension of 3.6 g (10.3 mmoles) of 1-[4'-(nitro)-styryl]-4-[methyl]-7,8-[methylenedioxy]-5H-2,3-benzodiazepine in 130 ml of methanol 17.7 ml (0.218 mole) of concentrated hydrogen chloride are added, under stirring. To the solution obtained in a few minutes 9.8 g (0.259 mole) of sodium borohydride are added in portions, within 30 minutes, and the mixture is stirred further for 30 minutes. Then 150 ml of distilled water is dropped to the orange suspension, the crude product is filtered off, washed four times with 20 ml each of distilled water and dried at a temperature between 80°C and 100°C. Thus 3.37 g of the desired product are obtained. In order to purify the crude product it is boiled with

17 ml of ethanol, cooled, filtered, washed and dried. Thus 2.67 g (73.8%) of the desired compound are obtained. M.p.: 175 to 177°C (decomp.).

The compounds of the following Examples 25 to 29 can be prepared according to the method of Example 24.

Example 25

1-[4'-(Nitro)-styryl]-4-[methyl]-7,8-di-[methoxy]-3,4-di-[hydro]-5H-2,3-benzodiazepine

Yield: 65,0%. M.p.: 173 to 175°C (decomp.) (Et0H).

Example 26

1-[4'-(Nitro)-styryl]-4-[methyl]-5-[ethyl]-7,8-di-[methoxy]-3,4-di-[hydro]-5H-2,3-benzodiazepine

Yield: 64.5%. M.p.: 168 to 169°C (decomp.) (Et0H).

Example 27

1-[Styryl]-4-[methyl]-5-[ethyl]-7,8-[methylenedioxy]-3,4-di-[hydro]-5H-2,3-benzodiazepine

One proceeds as specified in Example 24, with the difference that after the addition of the sodium borohydride and the completion of the reaction the mixture is evaporated, and the crude product solidified with water is recrystallized from ethanol.
Yield: 40%. M.p.: 153 to 154°C.

Example 28

1-[3',4'-Di-(chloro)-styryl]-4-[methyl]-7,8-di-[methoxy]-3,4-di-[hydro]-5H-2,3-benzodiazepine

One proceeds as specified in Example 24, and the reaction mixture is worked up according to Example 27.
Yield: 54.0%. M.p.: 132 to 133°C (Et0H).

Example 29

1-[3'-(Chloro)-styryl]-4-[methyl]-7,8-[methylenedioxy]-3,4-di-[hydro]-5H-2,3-benzodiazepine

One proceeds as specified in Example 24 and the reaction mixture is then worked up according to Example 27.
Yield: 40.0 e. M.p.: 114-117 OC (EtOH).

Example 30

1-[4'-(Nitro)-styryl]-3-[acetyl]-4-[methyl]-7,8-di-[methoxy]-3,4-di-[hydro]-5H-2,3-benzodiazepine

2.6 g (7.07 mmoles) Of the compound prepared according to Example 25 are stirred with 13 ml of acetic anhydride at 25°C for 1 hour, 50 ml of distilled water are added to it and the mixture is stirred further for 1 hour. The separated yellow precipitate is filtered off, washed three times with 15 ml each of distilled water and dried at a temperature between 80°C and 100°C. Thus 2.68 g of crude product are obtained, which is recrystallized from 13 ml of hot ethanol. Thus 2.62 g (90.6%) of the desired product are obtained in pure form.
M.p.: 182 to 184°C.

The compounds of the following Examples 31 and 32 are prepared according to the method specified in Example 30.

Example 31

1-[4'-(Nitro)-styryl]-3-[acetyl]-4-[methyl]-7,8-[methylenedioxy]-3,4-di-[hydro]-5H-2,3-benzodiazepine

Yield: 91.0%. M.p.: 188 to 190°C (Et0H).

Example 32

1-[4'-(Nitro)-styryl]-3-[acetyl]-4-[methyl]-5-[ethyl]-7,8-di-[methoxy]-3,4-di-[hydro]-5H-2,3-benzodiazepine

Yield: 88.0%. M.p.: 184 to 185°C.

Example 33

1-[4'-(Amino)-styryl]-4-[methyl]-7,8-di-[methoxy]-3,4-di-[hydro]-5H-2,3-benzodiazepine

6.95 g (18.9 mmoles) of 1-[4'-(nitro)-styryl]-4-[methyl]-7,8-di-[methoxy]-3,4-di-[hydro]-5H-2,3-benzodiazepine prepared according to Example 25 are suspended in 170 ml of methanol, 0.7 g of dry (corresponding to about 1.4 g of wet) Raney nickel catalyst and 3.3 ml (66 mmoles) of 100% by weight hydrazine hydrate are added to it and the reaction mixture is stirred for 1 hour. A solution is obtained, and in the beginning the inner temperature rises to 40 to 45°C. The catalyst is filtered off, washed three times with 15 ml each of methanol, the filtrate is evaporated in vacuo, the crude product is conveyed to a filter with 80 ml of water, washed three times with 15 ml each of water and dried. Thus 5.46 g of product are obtained. In order to purify the crude product it is recrystallized from 25 ml of 50% by volume ethanol. Thus 4.21 g (66.0%) of the desired product are obtained. M.p.: 152 to 154°C.

The compounds of the following Examples 34 to 38 can be prepared according to the method of Example 33.

Example 34

1-[4'-(Amino)-styryl]-4-[methyl]-7,8-[methylenedioxy]-3,4-di-[hydro]-5H-2,3-benzodiazepine

Yield: 80.0%. M.p.: 159 to 161°C (50% by volume Et0H).

Example 35

1-[4'-(Amino)-styryl]-4-[methyl]-5-[ethyl]-7,8-di-[methoxy]-3,4-di-[hydro]-5H-2,3-benzodiazepine

Yield: 75.5%. M.p.: 155 to 158°C (50% by volume Et0H).

Example 36

1-[4'-(Amino)-styryl]-3-[acetyl]-4-[methyl]-7,8-di-[methoxy]-3,4-di-[hydro]-5H-2,3-benzodiazepine

One proceeds as specified in Example 33, with the difference that owing to the hard solubilities of the starting compound and the end-product a mixture of dichloromethane and methanol in a ratio by volume of 2 : 1 is used as solvent. Yield: 81.4%. M.p.: 253 to 255°C (decomp.) (Et0H).

Example 37

1-[4'-(Amino)-styryl]-3-[acetyl]-4-[methyl]-7,8-[methylenedioxy]-3,4-di-[hydro]-5H-2,3-benzodiazepine

Yield: 68.9%. M.p.: 233 to 234°C (decomp.) (Et0H).

Example 38

1-[4'-(Amino)-styryl]-3-[acetyl]-4-[methyl]-5-[ethyl]-7,8-di-[methoxy]-3,4-di-[hydro]-5H-2,3-benzodiazepine

Yield: 77.1%. M.p.: 104 to 106°C (Et0H).

Example 39

1-[4'-(Acetylamino)-styryl]-3-[acetyl]-4-[methyl]-7,8-di-[methoxy]-3,4-di-[hydro]-5H-2,3-benzodiazepine

1.2 g (3.56 mmoles) of 1-[4'-(amino)-styryl]-4-[methyl]-7,8-di-[methoxy]-3,4-di-[hydro]-5H-2,3-benzodiazepine prepared according to Example 33 is suspended in 6 ml of acetic anhydride. The suspension is stirred for 1 hour at room temperature. In the meantime the starting compound gets dissolved, the end-product begins to separate and the reaction mixture gets thick. The separated product is filtered off, washed three times with 15 ml each of diethyl ether and dried at a temperature between 80°C and 100°C. Thus 1.07 g (71.3%) of the desired compound is obtained.
M.p.: 243 to 246°C (decomp.).

Example 39a

On starting from the compound of Example 36 and proceeding as specified in Example 39 the desired compound is obtained in a yield of 78%.

The compounds of the following Examples 40 and 41 can be prepared according to the method specified in Example 39.

Example 40

1-[4'-(Acetylamino)-styryl]-3-[acetyl]-4-[methyl]-7,8-[methylenedioxy]-3,4-di-[hydro]-5H-2,3-benzodiazepine

Yield: 91.0%. M.p.: 252 to 255°C (decomp.).

Example 41

1-[4'-(Acetylamino)-styryl]-3-[acetyl]-4-[methyl]-5-[ethyl]-7,8-[methylenedioxy]-3,4-di-[hydro]-5H-2,3-benzodiazepine

Yield: 73.5%. M.p.: 137 to 140°C (Et0H).

Example 42

1-[2'-(Naphth-1"-yl)-vinyl]-4-[methyl]-7,8-di-[methoxy]-3,4-di-[hydro]-5H-2,3-benzodiazepine

To a solution of 5.35 g (14.3 mmoles) of 1-[2'-naphth-1"-yl)-vinyl]-4-[methyl]-7,8-di-[methoxy]-5H-2,3-benzodiazepine in 30 ml of glacial acetic acid a solution of 1.76 g (46.3 mmoles) of sodium borohydride in 10 ml of water is dropped at 50°C, under stirring, and the reaction mixture is stirred further for 2 hours. The product is then stirred with 250 ml of water, made alkaline with concentrated ammonium hydroxide solution, the separated yellow precipitate is filtered, washed with water and recrystallized from isopropanol. Thus 3.75 g (70.5%) of the desired compound are obtained.
M.p.: 148 to 152°C.

The new starting compounds used for the preparation of the compounds of the above Examples are summarized the following Table VI. In this Me = methyl and Et = ethyl.

Table VI

| Starting compound for preparing compound (No. of Example) | $R_1$ | $R_2$ | $R_3$ | $R_4$ | M.p. (°C) |
|---|---|---|---|---|---|
| 1 | 3,4-di-(methoxy)--phenyl | H | - $CH_2$ - | | 206 to 208 |
| 2 | 3-(chloro)-phenyl | H | Me | Me | 178 to 180 |
| 3 | 3,4-(methylenedi-oxy)-phenyl | H | Me | Me | 158 to 160 |
| 4 | 4-(hydroxy)-phenyl | H | Me | Me | 212 to 213 |
| 5 | 3-(ethoxy)-4--(hydroxy)-phenyl | H | Me | Me | 120 to 122 |
| 6 | 2,4,6-tri--(methoxy)-phenyl | H | Me | Me | 191 to 193 |
| 7 | 4-(dimethylami-no)-phenyl | H | Me | Me | 170 to 172 |
| 8 | 3,4-(methylene-dioxy)-phenyl | H | - $CH_2$ - | | 223 to 225 |
| 9 | 4-(dimethylami-no)-phenyl | H | - $CH_2$ - | | 219 to 222 |
| 10 | 4-(dimethylami-no)-phenyl | H | Et | Et | 158 to 160 |
| 11 | 3-(hydroxy)--phenyl | H | Me | Me | 220 to 221 |
| 12 | phenyl | H | Me | Me | 143 to 145 |

Table VI continued

| Starting compound for preparing compound (No. of Example) | $R_1$ | $R_2$ | $R_3$ | $R_4$ | M.p. (°C) |
|---|---|---|---|---|---|
| 13 | 3-(isopropyl)--4-(methoxy)--phenyl | H | - $CH_2$ - | | 155 to 157 |
| 14 | 2-(bromo)-phenyl | H | Me | Me | 176 to 178 |
| 15 | 3,4-di-(methoxy)--phenyl | Et | Me | Me | 158 to 160 |
| 16 | 2,3-di-(methoxy)--phenyl | H | - $CH_2$ - | | 149 to 150 |
| 17 | 2,4-di-(methoxy)--phenyl | H | - $CH_2$ - | | 210 to 212 |
| 18 | 2,4-di-(methoxy)--phenyl | H | Me | Me | 151 to 153 |
| 24 | 4-(nitro)-phenyl | H | - $CH_2$ - | | 227 to 228 |
| 25 | 4-(nitro)-phenyl | H | Me | Me | 224 to 226 |
| 26 | 4-(nitro)-phenyl | Et | Me | Me | 218 to 220 |
| 27 | phenyl | H | - $CH_2$ - | | 153 to 154 |
| 28 | 3,4-di-(chloro)--phenyl | H | Me | Me | 168 to 169 |
| 29 | 3-(chloro)-phenyl | H | - $CH_2$ - | | 135 to 137 |
| 42 | naphth-1-yl | H | Me | Me | 148 to 152 |

## Claims

1. 1-[2'-(Substituted) vinyl]-3,4-di-[hydro]-5H-2,3-benzodiazepine derivatives of general formula

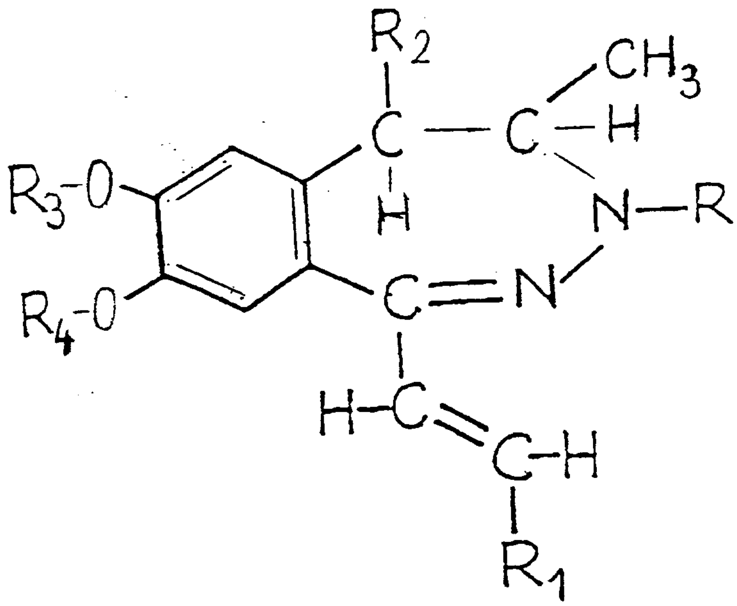

I,

wherein

| | |
|---|---|
| R | represents hydrogen or $C_{1-4}$-alkanoyl, |
| $R_1$ | stands for phenyl, optionally carrying 1 to 3 identical or different substituent(s) selected from the group consisting of halogen, nitro, amino, $C_{1-4}$-alkylamino, di-($C_{1-4}$-alkyl)-amino, $C_{1-4}$-alkanoylamino, $C_{1-4}$-alkyl, $C_{1-4}$-alkoxy, methylenedioxy and hydroxy; or naphthyl, optionally carrying a substituent selected from the group consisting of hydroxy, $C_{1-4}$-alkyl and $C_{1-4}$-alkoxy; |
| $R_2$ | stands for hydrogen or $C_{1-4}$-alkyl; |
| $R_3$ and $R_4$ | independently represent $C_{1-4}$-alkyl or |
| $R_3$ and $R_4$ | together form methylene, |

as well as acid addition salts and stereoisomers and mixtures thereof.

2. 1-[2'-(Substituted) vinyl]-3,4-di-[hydro]-5H-2,3-benzodiazepine derivatives according to claim 1, characterized in that the alkyl and/or alkoxy group(s) by which the phenyl group by which $R_1$ may be represented may be substituted or the alkyl or alkoxy group by which the naphthyl group by which $R_1$ may be represented may be substituted is/are such having from 1 to 3, particularly 1 or 2, carbon atom(s).

3. 1-[2'-(Substituted) vinyl]-3,4-di-[hydro]-5H-2,3-benzodiazepine derivatives according to claim 1 or 2, characterized in that the alkyl part(s) of the alkylamino and/or di-(alkyl)-amino group(s) by which the phenyl group by which $R_1$ may be represented may be substituted is/are such having from 1 to 3, particularly 1 or 2, carbon atom(s).

4. 1-[2'-(Substituted) vinyl]-3,4-di-[hydro]-5H-2,3-benzodiazepine derivatives according to claims 1 to 3, characterized in that the alkanoylamino group(s) by which the phenyl group by which $R_1$ may be represented may be substituted and/or which may be represented by R is/are such having from 1 to 3, particularly 2 or 3, carbon atom(s).

**5.** 1-[2'-(Substituted) vinyl]-3,4-di-[hydro]-5H-2,3-benzodiazepine derivatives according to claims 1 to 4, characterized in that the halogen atom(s) by which the phenyl group by which $R_1$ may be represented may be substituted is/are fluorine, bromine and/or chlorine.

**6.** 1-[2'-(Substituted) vinyl]-3,4-di-[hydro]-5H-2,3-benzodiazepine derivatives according to claims 1 to 5, characterized in that the alkyl group which may be represented by $R_2$ is such having from 1 to 3, particularly 1 or 2, carbon atom(s).

**7.** 1-[2'-(Substituted) vinyl]-3,4-di-[hydro]-5H-2,3-benzodiazepine derivatives according to claims 1 to 6, characterized in that the alkyl group(s) which may be represented by $R_3$ and/or $R_4$ is/are such having from 1 to 3, particularly 1 or 2, carbon atom(s).

**8.** 1-[2'-(Substituted) vinyl]-3,4-di-[hydro]-5H-2,3-benzodiazepine derivatives according to claims 1 to 7, characterized in that

| | |
|---|---|
| R | represents $C_{1-4}$-alkanoyl, |
| $R_1$ | stands for phenyl or naphthyl carrying a $C_{1-4}$-alkanoylamino or $C_{1-4}$-alkoxy substituent, |
| $R_2$ | stands for hydrogen or ethyl and |
| $R_3$ and $R_4$ | independently represent $C_{1-4}$-alkyl, |

as well as acid addition salts and stereoisomers and mixtures thereof.

**9.** 1-[4'-(Acetylamino)-styryl]-3-[acetyl]-4-[methyl]-7,8-di-[methoxy]-3,4-di-[hydro]-5H-2,3-benzodiazepine, 1-[2'-(naphth-1''-yl)-vinyl]-4-[methyl]-7,8-di-[methoxy]-3,4-di-[hydro]-5H-2,3-benzodiazepine and 1-[2',3'-di-(methoxy)-styryl]-3-[acetyl]-4-[methyl]-7,8-di-[methoxy]-3,4-di-[hydro]-5H-2,3-benzodiazepine as well as acid addition salts and stereoisomers and mixtures thereof.

**10.** A process for preparing the 1-[2'-(substituted) vinyl]-3,4-di-[hydro]-5H-2,3-benzodiazepine derivatives according to claims 1 to 9, characterized in that

a) reducing 1-[2'-(substituted) vinyl]-5H-2,3-benzodiazepine derivatives of general formula

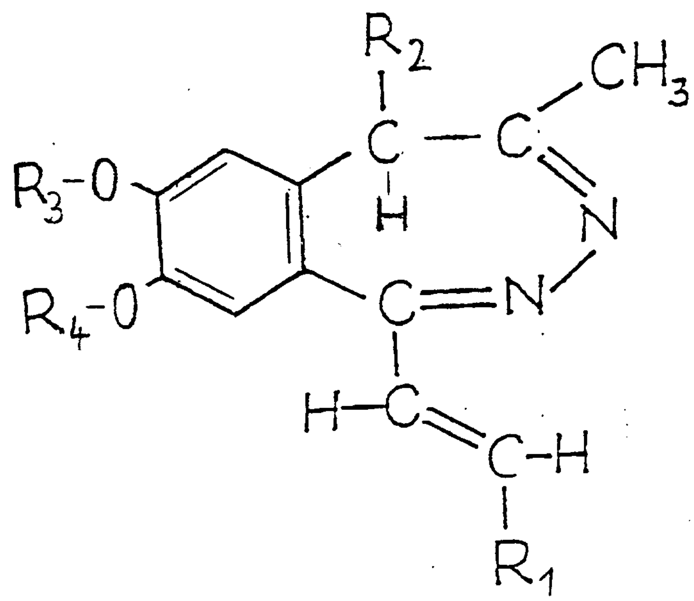

II,

wherein $R_1$ , $R_2$ , $R_3$ and $R_4$ are as defined in claims 1 to 8, with complex metal hydrides, boranes and/or borane complexes, and optionally in a manner known per se acylating the 1-[2'-(substituted) vinyl]-3,4-di-[hydro]-5H-2,3-benzodiazepine derivatives of general formula I thus obtained, wherein R stands for hydrogen and $R_1$ , $R_2$ , $R_3$ and $R_4$ are as defined in claims 1 to 8, or

b) in a manner known per se for preparing 1-[2'-(substituted) vinyl]-3,4-di-[hydro]-5H-2,3-benzodiazepine derivatives of general formula I, wherein $R_1$ represents aminophenyl, ($C_{1-4}$-alkyl)-aminophenyl, di-($C_{1-4}$-alkyl)-aminophenyl or ($C_{1-4}$-alkanoyl)-aminophenyl, which groups optionally carry 1 or 2 identical or different substituent(s) selected from the group consisting of halogen, nitro, amino, $C_{1-4}$-alkyl, $C_{1-4}$-alkoxy, methylenedioxy and hydroxy, and R, $R_2$ , $R_3$ and $R_4$ are as defined in claims 1 to 8, reducing 1-[2'-(substituted) vinyl]-3,4-di-[hydro]-5H-2,3-benzodiazepine derivatives of general formula I, wherein $R_1$ represents nitrophenyl, optionally carrying 1 or 2 identical or different substituent(s) selected from the group consisting of halogen, nitro, amino, methylenedioxy, $C_{1-4}$-alkyl, $C_{1-4}$-alkoxy and hydroxy, and R, $R_2$ , $R_3$ and $R_4$ are as defined in claims 1 to 8, with hydrazine hydrate in the presence of a catalyst, and optionally acylating or alkylating the thus obtained 1-[2'-(substituted) vinyl]-3,4-di-[hydro]-5H-2,3-bezodiazepine derivatives of general formula I, wherein $R_1$ stands for aminophenyl and R, $R_2$ , $R_3$ and $R_4$ are as defined in claims 1 to 8,

and optionally in a manner known per se converting the 1-[2'-(substituted) vinyl]-3,4-di-[hydro]-5H-2,3-benzodiazepine derivatives of general formula I obtained by any of the above variants a) and b) into acid addition salts thereof and/or optionally converting the acid addition salts of 1-[2'-(substituted) vinyl]-3,4-di-[hydro]-5H-2,3-benzodiazepine derivatives of general formula I obtained by any of the above variants a) and b) into the corresponding free 1-[2'-(substituted) vinyl]-3,4-di-[hydro]-5H-2,3-benzodiazepine derivatives of general formula I and/or into other acid addition salts and/or resolving stereoisomer mixtures of the 1-[2'-(substituted) vinyl]-3,4-di-[hydro]-5H-2,3-benzodiazepine derivatives of formula I or acid addition salts thereof into their stereoisomers or converting the stereoisomers into mixtures thereof.

**11.** A process according to claim 10 a), characterized by carrying out the reduction of the 1-[2'-(substituted) vinyl]-5H-2,3-benzodiazepine derivatives of general formula II with complex metal hydrides, boranes and/or borane complexes in [an] organic solvent(s).

**12.** A process according to claim 10 a) or 11, characterized by carrying out the reduction of the 1-[2'-(substituted) vinyl]-5H-2,3-benzodiazepine derivatives of general formula II with complex metal hydrides, boranes and/or borane complexes in a solvent or solvent mixture, which does not interact with the applied reducing agent, or only very slowly.

**13.** A process according to claim 10 a) or 11 or 12, characterized by using in the reduction of the 1-[2'-(substituted) vinyl]-5H-2,3-benzodiazepine derivatives of general formula II with complex metal hydrides, boranes and/or borane complexes as reducing agent(s) sodium borohydride, lithium aluminum hydride, [a] borane(s) or [a] borane-trimethylamine complex(es).

**14.** A process according to claim 10 b), characterized by using in the reduction of the 1-[2'-(substituted) vinyl]-3,4-di-[hydro]-5H-2,3-benzodiazepine derivatives of general formula I with hydrazine hydrate as catalyst palladium, platinum or Raney nickel.

**15.** A process according to claim 10 b) or 14, characterized by carrying out the reduction of the 1-[2'-(substituted) vinyl]-3,4-di-[hydro]-5H-2,3-benzodiazepine derivatives of general formula I with hydrazine hydrate in [an] organic solvent(s).

**16.** A process according to claim 10 b) or 14 or 15, characterized by using in the reduction of the 1-[2'-(substituted) vinyl]-3,4-di-[hydro]-5H-2,3-benzodiazepine derivatives of general formula I with hydrazine hydrate as [a] solvent(s) [a] $C_{1-4}$-alcohol(s), dioxane, tetrahydrofurane, benzene, chloroform, dimethylformamide or dichloromethane, dimethyl sulfoxide or pyridine or mixtures thereof.

**17.** A process according to claims 10 to 16, characterized by carrying out the reduction of the 1-[2'-(substituted) vinyl]-5H-2,3-benzodiazepine derivatives of general formula II with complex metal hydrides, boranes and/or borane complexes or of the 1-[2'-(substituted) vinyl]-3,4-di-[hydro]-5H-2,3-benzodiazepine derivatives of general formula I with hydrazine hydrate at a temperature of from 0°C to the boiling point of the applied solvent or solvent mixture, particularly at a temperature of from +10 to 100°C.

**18.** Medicaments, characterized in that they contain 1 or more 1-[2'-(substituted) vinyl]-3,4-di-[hydro]-5H-2,3-di-benzodiazepine derivative(s) according to claims 1 to 9 as [an] active ingredient(s), advantageously in admixture with 1 or more inert solid and/or liquid pharmaceutical carrier(s) and/or auxiliari(es).

**19.** Use of the 1-[2'-(substituted) vinyl]-3,4-di-[hydro]-5H-2,3-benzodiazepine derivatives according to claims 1 to 9 for preparing medicaments, particularly for the treatment of central nervous system disorders.

European Patent Office

# EUROPEAN SEARCH REPORT

Application Number
EP 96 10 1883

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | FR-A-2 501 686 (EGYT GYOGYSZERVEGYESZETI GYAR)<br>* the whole document *<br>--- | 1-19 | C07D243/02<br>C07D491/04<br>C07D405/06<br>A61K31/55 |
| A | FR-A-2 439 189 (EGYT GYOGYSZERVEGYESZETI GYAR)<br>* the whole document *<br>--- | 1-19 | |
| D,A | GB-A-2 190 677 (EGIS GYOGYSZERGYAR)<br>* the whole document *<br>--- | 1-19 | |
| A | FR-A-2 081 426 (LES LABORATOIRES S.M.B.)<br>* the whole document, particularly example 32 *<br>----- | 1-19 | |
| | | | TECHNICAL FIELDS SEARCHED (Int.Cl.6) |
| | | | C07D |

The present search report has been drawn up for all claims

| Place of search | Date of completion of the search | Examiner |
|---|---|---|
| THE HAGUE | 15 May 1996 | Allard, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)